# EUROPEAN PATENT APPLICATION

(11) **EP 3 677 655 A1**
(43) Date of publication of application: **08.07.2020**
(21) Application number: 18850058.1
(22) Date of filing: 31.07.2018
(51) Int. Cl.: C09K 3/00, A61K 8/02, A61K 8/36, A61K 8/58, A61K 8/81, A61Q 17/04

(54) **THICKENER FOR NON-AQUEOUS SYSTEMS, AND THICKENER COMPOSITION**

(30) Priority: 28.08.2017 JP 2017163567
(71) Applicant: Shiseido Company, Ltd., Chuo-ku Tokyo 104-8010 (JP)
(72) Inventor: NAGAI Kouichi, Yokohama-shi Kanagawa 224-8558 (JP); GOMI Hiroki, Osaka-shi Osaka 533-0004 (JP); NAOI Kayoko, Yokohama-shi Kanagawa 224-8558 (JP); NAGARE Yuko, Yokohama-shi Kanagawa 224-8558 (JP)
(74) Representative: Ter Meer Steinmeister & Partner
(86) International application number: PCT/JP2018/028543
(87) International publication number: WO 2019/044327

(57) **Abstract**

A thickener for a non-aqueous system includes a water-soluble polymer and a counterpart that ionically and/or electrostatically interacts with the water-soluble polymer.

## Description

### Cross Reference to Related Applications

The present invention is based upon and claims the benefit of the priority of Japanese Patent Application No. 2017-163567 (filed on August 28, 2017), the disclosure of which is incorporated herein in its entirety by reference.

### Technical Field

The present disclosure relates to a thickener for a non-aqueous system. The present disclosure also relates to a composition including the thickener.

### Background Art

Cosmetics mainly including a non-aqueous system solvent are known in the art (see, for example, Patent Literature 1). A sun-block cosmetic disclosed in Patent Literature 1 includes: from 0.05 to 10.0 wt% of a cationic thickener obtained by polymerizing a thickener monomer composition having a specific structure; from 0.01 to 20.0 wt% of a UV absorber; and from 30.0 to 90.0 wt% of ethanol. The cosmetic further includes powder.

### Citation List

### Patent Literature

Patent Literature 1: Japanese Unexamined Patent Publication No. H8-268857A

### Summary of Invention

### Technical Problem

In the sun-block cosmetic disclosed in Patent Literature 1, the powder acts as a UV scattering agent. Unfortunately, cosmetics including powder have such drawbacks as that, when the cosmetic is applied to the skin, the powder tends to leave a white cast, and may cause unevenness in color. These phenomena are not favored by users. In recent years, users tend to favor cosmetics having a highly transparent appearance. Unfortunately, cosmetics including powder are usually opaque. Thus, there are demands for cosmetics with reduced amounts of powder.

For example, in cases of reducing the amount of powder in a sun-block cosmetic, a UV absorber, which is an organic compound, is added in place of the powder to maintain UV-blocking effects. UV absorbers are basically oil-soluble; thus, cosmetics including UV absorbers need to employ a non-aqueous system solvent, such as an organic solvent, as a main solvent. On the other hand, the viscosity of the organic solvent makes it inconvenient to apply the cosmetic to the skin. Thus, to improve the usability of the cosmetic, there is a need to make the viscosity of the cosmetic higher than the viscosity of the organic solvent. Unfortunately, there is no thickener suitable for non-aqueous system solvents; in order to thicken a cosmetic mainly including a non-aqueous system solvent, there is a need to create a water-in-oil cosmetic by emulsifying a thickened aqueous system in a non-aqueous system. A water-in-oil system, however, has reduced transparency due to its emulsion particles, making it impossible to achieve a highly-transparent cosmetic.

Thus, there is a demand for a thickener capable of thickening non-aqueous system solvents without employing emulsification. There is also a demand for a thickened composition that is highly transparent and has excellent usability, despite mainly including a non-aqueous system solvent.

### Solution to Problem

According to a first aspect of the present disclosure, a thickener for a non-aqueous system is provided, the thickener comprising a water-soluble polymer and a counterpart that ionically and/or electrostatically interacts with the water-soluble polymer.

According to a second aspect of the present disclosure, a thickened composition is provided, the composition comprising the thickener for a non-aqueous system according to the first aspect and a polar solvent capable of dissolving the water-soluble polymer and the counterpart.

### Advantageous Effects of Invention

The thickener of the present disclosure can thicken compositions in which the solvent is mainly a non-aqueous system. With the thickener of the present disclosure, a highly-transparent thickened composition can be obtained.

With the composition of the present disclosure, it is possible to achieve high transparency and a viscosity offering excellent usability, despite including oil-soluble components.

### Brief Description of Drawings

[Fig. 1] Fig. 1 is a graph showing a relationship between viscosity and the counterpart admixture amount in Test Examples 1 to 6.
[Fig. 2] Fig. 2 is a graph showing a relationship between viscosity and the content by percentage of a polar solvent in Test Examples 26 to 29.
[Fig. 3] Fig. 3 is a graph showing a relationship between viscosity and the water admixture percentage in Test Examples 30 to 35.

### Description of Embodiments

Preferred modes of the aforementioned aspects are described below.

According to a prefered mode of the above first aspect, the non-aqueous system includes a polar organic solvent as a main solvent.

According to a prefered mode of the above first aspect, the water-soluble polymer includes a first polar group. The counterpart includes a second polar group. One of the first polar group and the second polar group is an anionic functional group, and the other is a cationic functional group.

According to a prefered mode of the above first aspect, the first polar group and the second polar group are each either a carboxy group or an amine.

According to a prefered mode of the above first aspect, the water-soluble polymer further includes a heterocycle.

According to a prefered mode of the above first aspect, the heterocycle is a lactam.

According to a prefered mode of the above first aspect, the water-soluble polymer is a copolymer of a first component including the first polar group and a second component including the heterocycle.

According to a prefered mode of the above first aspect, the first component is a 2-(dimethylamino)ethyl methacrylate component. The second component is a vinylpyrrolidone component.

According to a prefered mode of the above first aspect, the water-soluble polymer is a copolymer further including a cross-linking third component.

According to a prefered mode of the above second aspect, the water-soluble polymer is a copolymer further including a fourth component including an alkyl acrylate and/or an acrylamide.

According to a prefered mode of the above first aspect, in the water-soluble polymer, with respect to the total amount of the first to fourth components, the proportion of the first component is from 15 to 85% by mass. The proportion of the second component is from 20 to 80% by mass. Tthe proportion of the third component is from 0 to 20% by mass. The proportion of the fourth component is from 0 to 60% by mass.

According to a prefered mode of the above first aspect, the water-soluble polymer is a vinyl pyrrolidone/N,N-dimethylaminoethyl methacrylate/stearyl acrylate/tripropylene glycol diacrylate copolymer.

According to a prefered mode of the above first aspect, the counterpart is an anionic organic compound.

According to a prefered mode of the above first aspect, the number of carbon atoms in the counterpart is from 3 to 24.

According to a prefered mode of the above first aspect, the counterpart is at least one selected from the group consisting of isostearic acid, lactic acid, lauric acid, myristic acid, palmitic acid, stearic acid, 12-hydroxystearic acid, behenic acid, and 3-(10-carboxydecyl)-1,1,1,3,5,5,5-heptamethyltrisiloxane.

According to a prefered mode of the above first aspect, the water-soluble polymer includes an acidic functional group.

According to a prefered mode of the above first aspect, the water-soluble polymer is a copolymer of acrylic acid and an alkyl acrylate.

According to a prefered mode of the above first aspect, the water-soluble polymer is an acrylates/C10-30 alkyl acrylate crosspolymer.

According to a prefered mode of the above first aspect, the counterpart is a cationic organic compound.

According to a prefered mode of the above first aspect, the counterpart is distearyldimonium chloride.

According to a prefered mode of the above first aspect, the water-soluble polymer and the counterpart form an ion pair.

According to a prefered mode of the above second aspect, [Claim 22] The thickener according to any one of claims 1 to 21, wherein the content ratio of the counterpart is from 0.02 to 10 parts by mass with respect to 1 part by mass of the water-soluble polymer.

According to a prefered mode of the above first aspect, the thickener is present in a polar solvent.

According to a prefered mode of the above second aspect, the content by percentage of the thickener is from 0.1 to 15% by mass relative to the mass of the composition.

According to a prefered mode of the above second aspect, the polar solvent is an organic solvent.

According to a prefered mode of the above second aspect, the polar solvent is at least one selected from the group consisting of ethanol, dipropylene glycol, butylene glycol, glycerin, polyethylene glycol, polypropylene glycol, octyl methoxycinnamate, octocrylene, homosalate, and octyl salicylate.

According to a prefered mode of the above second aspect, the content by percentage of the polar solvent is 20% by mass or greater relative to the mass of the composition.

According to a prefered mode of the above second aspect, the composition further comprises an oily component having mutual solubility to the polar solvent.

According to a prefered mode of the above second aspect, the oily component is at least one selected from the group of consisting of hydrocarbon oils, ester oils, and silicone oils.

According to a prefered mode of the above second aspect, the content by percentage of the oily component is 85% by mass or less relative to the mass of the composition.

According to a prefered mode of the above second aspect, in a case where the composition includes water, the content by percentage of water is 10% by mass or less relative to the polar solvent.

According to a prefered mode of the above second aspect, the composition further comprises a UV absorber.

According to a prefered mode of the above second aspect, the UV absorber is soluble in the oily component and/or the polar solvent.

According to a prefered mode of the above second aspect, the L* value in Lab color space is 90 or greater.

According to a prefered mode of the above second aspect, the viscosity is from 300 to 500,000 mPa·s.

In the following description, POE is an abbreviation of polyoxyethylene, and POP is an abbreviation of polyoxypropylene. The number in parentheses after POE or POP indicates the average number of moles of POE groups or POP groups added in the compound in question.

In the description below, a component (monomer) constituting a copolymer may include derivatives thereof. For example, an acrylic acid component may include an acrylic acid derivative. In the description below, "(meth)acrylic acid ((meth)acrylate)" refers to acrylic acid (acrylate) and/or methacrylic acid (methacrylate).

A thickener according to a first embodiment of the present disclosure will be described. The thickener of the present disclosure is capable of thickening non-aqueous systems and aqueous systems. In the present disclosure, a "non-aqueous system" refers to a composition in which the main solvent is a non-aqueous system solvent. A "non-aqueous system solvent" is a solvent other than water, and may be, for example, an organic solvent (including alcohol). Particularly, the non-aqueous system solvent may preferably be a polar solvent, as described in the second embodiment. The non-aqueous system may include water, and does not intend to exclude solvents including water.

The thickener of the present disclosure includes a water-soluble polymer and a counterpart.

### {Water-Soluble Polymer}

It is preferred that the water-soluble polymer is soluble in a polar solvent, such as water or alcohol. The water-soluble polymer may be, for example, an ionic polymer. The water-soluble polymer may be a polymer that ionizes in the presence of an acid or a base. For the ionic polymer, it is possible to select, for example, at least one selected from cationic polymers, anionic polymers, and amphoteric polymers.

Preferably, the water-soluble polymer includes a hydrophobic moiety (hydrophobic group). It is considered that the presence of a hydrophobic moiety can make the water-soluble polymer soluble (at least in part) in non-aqueous system solvents and oily components, thereby achieving a thickening effect to non-aqueous system solvents and oily components. The hydrophobic moiety may be, for example, at least one selected from later-described first, fourth, and sixth components.

The water-soluble polymer interacts in an ionic and/or electrostatic manner with the later-described counterpart. It is considered, for example, that the water-soluble polymer bonds ionically and/or electrostatically to the counterpart. Preferably, the water-soluble polymer includes a first polar group in at least a portion thereof. It is considered that the first polar group interacts with a second polar group in the counterpart. The first polar group contributes to rendering the water-soluble polar polymer water-soluble. The first polar group may be ionic, i.e., may be a cationic functional group or an anionic functional group. The first polar group may be a basic functional group or an acidic functional group. The first polar group may be a functional group that ionizes in the presence of an acid or a base.

Examples of the first polar group may include a carboxy group, an amino group, secondary amines, tertiary amines, quaternary amines, betaine structures, and heterocycles. Preferably, the first polar group is provided at a terminal of a side chain.

A first example of the water-soluble polymer will be described. The water-soluble polymer according to the first example is a cationic/basic polymer.

The water-soluble polymer may further include a heterocycle. Examples of the heterocycle may include lactams (cyclic amides). Examples of lactams may include three-membered rings, four-membered rings, five-membered rings (pyrrolidones), and six-membered rings (piperidones). Preferably, the heterocycle is provided at a terminal of a side chain.

The water-soluble polymer may be a copolymer of a first component including the first polar group and a second component including the heterocycle.

For example, the first component may include a structure represented by the following Chem. 1. In Chem. 1, R¹ represents a hydrogen atom or a methyl group; R² and R³ each independently represent a hydrogen atom, a methyl group, an ethyl group, or a t-butyl group; A represents an oxygen atom or a NH group; and B represents a C₁₋₄ alkylene group that is linear or that includes a side chain.

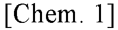

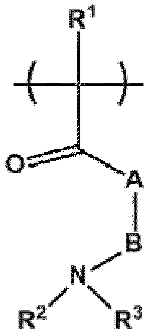

For example, the first component may be a (meth)acrylic acid derivative including an amine. Examples of the first component may include 2-(dimethylamino)ethyl (meth)acrylate, 3-(dimethylamino)propyl (meth)acrylate, N-[2-(dimethylamino)ethyl] (meth)acrylamide, and N-[3-(dimethylamino)propyl] (meth)acrylamide. The first component may be a single type of component, or a combination of a plurality of types of components.

For example, the second component may include a structure represented by the following Chem. 2 or Chem. 3. In Chem. 2 and Chem. 3, R⁴ represents a hydrogen atom or a methyl group. In Chem. 2, p represents 3 or 4.

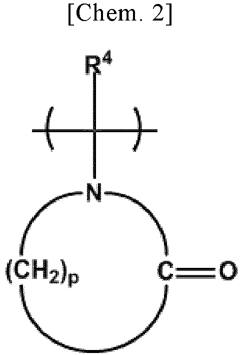

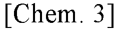

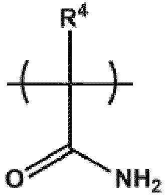

Examples of the heterocycle in the second component may include lactams (cyclic amides). Examples of lactams may include three-membered rings, four-membered rings, five-membered rings (pyrrolidones), and six-membered rings (piperidones). Preferably, the heterocycle is provided at a terminal of a side chain.

Examples of the second component may include N-vinylpyrrolidone, N-vinylpiperidone, acrylamide components, and methacrylamide components. The second component may be a single type of component, or a combination of a plurality of types of components.

Preferably, the water-soluble polymer is a copolymer further including a cross-linking third component. For the third component, it is possible to use, for example, a compound including two or more carbon-carbon unsaturated double bonds in a single molecule. For example, the third component may be a monomer having vinyl groups at both terminals.

For example, the third component may include a structure represented by the following Chem. 4. In Chem. 4, R⁵ represents a hydrogen atom or a methyl group; D represents an oxygen atom or a NH group; R⁶ represents a C₁₋₁₇ alkylene group that is linear or that includes a side chain, or a group represented by Chem. 5; and R⁷ represents a hydrogen atom or a methyl group. In Chem. 5, n represents an integer from 1 to 4; and q represents an integer from 1 to 25.

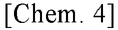

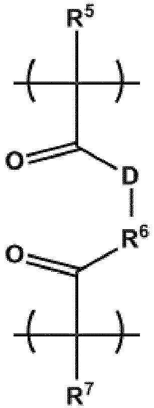

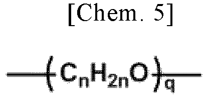

Preferably, the third component includes a hydrophilic moiety. The hydrophilic moiety is preferably located between two double bonds that contribute to polymerization. For example, the hydrophilic moiety may be a polyoxyalkylene chain, such as a polyoxyethylene chain or a polyoxypropylene chain. The average number of moles of polyoxyethylene groups and/or polyoxypropylene groups added may be, for example, one or greater, preferably two or greater. The average number of moles of polyoxyethylene groups and/or polyoxypropylene groups added may be, for example, ten or less, preferably five or less.

Examples of the third component may include (poly)ethylene glycol di(meth)acrylate, (poly)propylene glycol di(meth)acrylate, trimethylolpropane tri(meth)acrylate, pentaerythritol tri(meth)acrylate, methylene-bis-(meth)acrylamide, 1,2-bis-(meth)acrylamidoethane, 1,5-bis-(meth)acrylamidopentane, and divinylbenzene. The third component may be a single type of component, or a combination of a plurality of types of components.

Preferably, the water-soluble polymer is a copolymer further including a fourth component including a (meth)acryloyl group.

For example, the fourth component may include a structure represented by the following Chem. 6. In Chem. 6, R⁸ represents a hydrogen atom or a methyl group; E represents an oxygen atom or a NH group; R⁹ represents a C₁₋₁₇ alkylene group that is linear or that includes a side chain, or a group represented by Chem. 7; and R¹⁰ represents a hydrogen atom or a methyl group. In Chem. 7, n represents an integer from 1 to 4; and r represents an integer from 1 to 25.

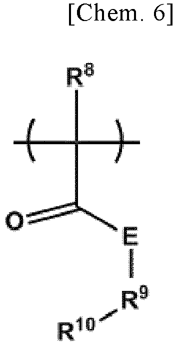

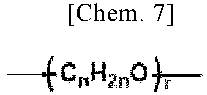

The fourth component may include a (meth)acryloyl group. Examples of the fourth component may include alkyl (meth)acrylates and alkyl (meth)acrylamides. The number of carbon atoms in the alkyl group may be, for example, one or greater, five or greater, eight or greater, or ten or greater. The number of carbon atoms in the alkyl group may be, for example, 50 or less, 40 or less, 30 or less, or 20 or less. Examples of the fourth component may include methyl (meth)acrylate, ethyl (meth)acrylate, n-propyl (meth)acrylate, isopropyl (meth)acrylate, n-butyl (meth)acrylate, isobutyl (meth)acrylate, t-butyl (meth)acrylate, n-hexyl (meth)acrylate, 2-ethylhexyl (meth)acrylate, octyl (meth)acrylate, lauryl (meth)acrylate, tridecyl (meth)acrylate, stearyl (meth)acrylate, cyclohexyl (meth)acrylate, N-t-butyl (meth)acrylamide, N-t-octyl (meth)acrylamide, hydroxyethyl (meth)acrylate, hydroxypropyl (meth)acrylate, and hydroxybutyl (meth)acrylate. The fourth component may be a single type of component, or a combination of a plurality of types of components.

The content by percentage of the first component, with respect to the total amount of the first to fourth components, is preferably 15% by mass or greater, more preferably 25% by mass or greater, even more preferably 35% by mass or greater. If the content by percentage is less than 15% by mass, the moiety that counters the counterpart will become small. The content by percentage of the first component, with respect to the total amount of the first to fourth components, is preferably 85% by mass or less, more preferably 75% by mass or less, even more preferably 65% by mass or less, even more preferably 55% by mass or less. If the content by percentage exceeds 85% by mass, solubility to oily components will deteriorate.

The content by percentage of the second component, with respect to the total amount of the first to fourth components, is preferably 20% by mass or greater, more preferably 30% by mass or greater, even more preferably 40% by mass or greater. If the content by percentage is less than 20% by mass, solubility to oily components will deteriorate. The content by percentage of the second component, with respect to the total amount of the first to fourth components, is preferably 80% by mass or less, more preferably 70% by mass or less, even more preferably 60% by mass or less, even more preferably 50% by mass or less. If the content by percentage exceeds 80% by mass, solubility to polar solvents will deteriorate.

The content by percentage of the third component, with respect to the total amount of the first to fourth components, may be 0% by mass, and is preferably 0.1% by mass or greater, more preferably 1% by mass or greater, even more preferably 3% by mass or greater, even more preferably 4% by mass or greater. The content by percentage of the third component, with respect to the total amount of the first to fourth components, is preferably 20% by mass or less, more preferably 15% by mass or less, even more preferably 10% by mass or less, even more preferably 8% by mass or less. If the content by percentage exceeds 20% by mass, aggregates are likely to be formed, making it difficult to obtain a uniform composition.

The content by percentage of the fourth component, with respect to the total amount of the first to fourth components, may be 0% by mass, and is preferably 0.5% by mass or greater, more preferably 1% by mass or greater, even more preferably 1.2% by mass or greater. The content by percentage of the fourth component, with respect to the total amount of the first to fourth components, is preferably 60% by mass or less, more preferably 30% by mass or less, more preferably 10% by mass or less, even more preferably 5% by mass or less. If the content by percentage exceeds 60% by mass, solubility to polar solvents will deteriorate.

The water-soluble polymer may include components other than the first to fourth components.

For the water-soluble polymer according to the first example, it is possible to use, for example, a vinyl pyrrolidone/N,N'-dimethylaminoethyl methacrylate/stearyl acrylate/tripropylene glycol diacrylate copolymer (CG polymer or Cosquat GA468 from Osaka Organic Chemical Industry Ltd.).

A second example of the water-soluble polymer will be described. The water-soluble polymer according to the second example is an anionic/acidic polymer.

The water-soluble polymer may be a (co)polymer including a fifth component including a (meth)acrylic acid component.

The water-soluble polymer may be a copolymer further including a sixth component including a (meth)acrylic acid derivative. Examples of the (meth)acrylic acid derivative may include alkyl (meth)acrylates. The number of carbon atoms in the alkyl group may be, for example, 1 or greater, 5 or greater, 8 or greater, or 10 or greater. The number of carbon atoms in the alkyl group may be, for example, 50 or less, 40 or less, 30 or less, or 20 or less.

An example of the water-soluble polymer according to the second example may include an acrylates/C 10-30 alkyl acrylate crosspolymer (PEMULEN (registered trademark) from Lubrizol Advanced Materials, Inc.).

The weight-average molecular weight of the water-soluble polymer is preferably 100,000 or greater, more preferably 500,000 or greater, even more preferably 1,000,000 or greater. If the weight-average molecular weight is less than 100,000, it becomes difficult to suppress aggregation of powder. The weight-average molecular weight of the water-soluble polymer is preferably 5,000,000 or less, more preferably 3,000,000 or less, even more preferably 2,000,000 or less. If the weight-average molecular weight exceeds 5,000,000, clogging and stickiness may arise. The weight-average molecular weight of the water-soluble polymer can be measured by employing gel permeation chromatography with light scattering (GPC-MALS).

### {Counterpart}

The counterpart is a compound that ionically and/or electrostatically interacts with the water-soluble polymer. It is considered that, by this interaction, the water-soluble polymer and the counterpart form a complex. Preferably, the counterpart is soluble in a polar solvent, such as water or alcohol. It is considered that the counterpart is ionically and/or electrostatically bondable to the first polar group of the water-soluble polymer.

Preferably, the counterpart includes a second polar group. It is considered that the second polar group interacts ionically and/or electrostatically with the first polar group. The second polar group may be an ionic functional group, i.e., a cationic functional group or an anionic functional group. The second polar group may be a basic functional group or an acidic functional group. The second polar group may be a functional group that ionizes in the presence of an acid or a base.

It is preferred that, when the first polar group and the second polar group ionize in water, the second polar group is a functional group having the opposite electric charge from the electric charge of the first polar group. For example, in cases where the first polar group in the water-soluble polymer is a cationic/basic functional group such as an amine, it is preferred that the second polar group in the counterpart is an anionic/acidic functional group such as a carboxy group. In this case, the counterpart may be, for example, an organic acid. In cases where the first polar group in the water-soluble polymer is an anionic/acidic functional group such as a carboxy group, it is preferred that the second polar group in the counterpart is a cationic/basic functional group such as an amine. In this case, the counterpart may be, for example, an organic base.

Examples of the second polar group may include a carboxy group, an amino group, secondary amines, tertiary amines, quaternary amines, betaine structures, and heterocycles.

Preferably, the counterpart is selected as appropriate depending on the solvent to be thickened. It is possible to adjust the solubility to polar solvents and oily components in accordance with the number of carbon atoms in the counterpart. The number of carbon atoms in the counterpart may be, for example, 3 or greater, 5 or greater, 8 or greater, 12 or greater, or 15 or greater. The number of carbon atoms in the counterpart may be, for example, 24 or less, or 20 or less. The counterpart may include a carbon chain (straight chain) in which the number of carbon atoms is 6 or greater, 8 or greater, 10 or greater, 12 or greater, 14 or greater, 16 or greater, 18 or greater, or 20 or greater.

In cases where the first polar group is cationic/basic, for the counterpart, it is possible to use, for example, lactic acid, a fatty acid, and/or a derivative thereof. Examples of fatty acids and derivatives thereof that may be used may include at least one selected from isostearic acid, lauric acid, myristic acid, palmitic acid, stearic acid, 12-hydroxystearic acid, behenic acid, and 3-(10-carboxydecyl)-1,1,1,3,5,5,5-heptamethyltrisiloxane.

In cases where the first polar group is anionic/acidic, for the counterpart, it is possible to use, for example, a primary amine, a secondary amine, a tertiary amine, or a quaternary amine. An example of a quaternary amine that may be used may include distearyldimonium chloride.

The content ratio of the counterpart in the thickener, with respect to 1 part by mass of the water-soluble polymer, is preferably 0.02 parts by mass or greater, more preferably 0.1 parts by mass or greater, even more preferably 0.2 parts by mass or greater, even more preferably 0.5 parts by mass or greater, further more preferably 1 part by mass or greater. Viscosity can be increased when the content ratio of the counterpart is 0.02 parts by mass or greater. The content ratio of the counterpart, with respect to 1 part by mass of the water-soluble polymer, may be 10 parts by mass or less, 8 parts by mass or less, 6 parts by mass or less, or 4 parts by mass or less. The content ratio of the counterpart can be determined as appropriate depending on the desired viscosity.

The thickener of the present disclosure may be formed or preserved in a polar solvent. The polar solvent will be described in a second embodiment.

In the thickener of the present disclosure, it is considered that the water-soluble polymer and the counterpart interact ionically and/or electrostatically in a solvent. There is a possibility that at least a portion of the first polar group and the second polar group is ionized in water which is present in the polar solvent. It is thus considered that the first polar group of the water-soluble polymer and the second polar group of the counterpart ionically and/or electrostatically bond with one another. It is considered that the water-soluble polymer and the counterpart form an ion pair (coupling). There is also a possibility that a plurality of water-soluble polymers and counterparts are linked and/or cross-linked two-dimensionally and/or three-dimensionally.

The thickener of the present disclosure is capable of thickening polar solvents such as alcohols and polyols. Particularly, the present thickener is capable of thickening organic polar solvents. Using the thickener of the present disclosure can thicken compositions including water-insoluble (oil-soluble) components. For example, it is possible to thicken a composition in which a water-insoluble component is dissolved, by: dissolving the water-insoluble component in an oily solvent having mutual solubility to a polar solvent; and thickening a mixture obtained by mixing the oily solvent and the polar solvent. Thus, it is possible to increase the viscosity, even of compositions mainly including a non-aqueous system solvent or an oily component, and thereby improve product usability.

It is considered that the thickening effect by the thickener of the present disclosure is not caused by emulsification. Thus, a composition thickened by the thickener of the present disclosure can be provided with high transparency. For example, the thickener of the present disclosure can thicken, while maintaining high transparency, a non-aqueous cosmetic in which an oil-soluble active component, such as a UV absorber, is dissolved.

The thickener of the present disclosure can be manufactured according to known methods, without being limited to a specific method. For example, the thickener of the present disclosure can be manufactured by mixing the aforementioned components in a polar solvent.

A composition according to a second embodiment of the present disclosure will be described. The composition of the present disclosure is applicable, for example, to cosmetics (e.g., sun-block cosmetics).

The composition of the present disclosure includes a water-soluble polymer, a counterpart, and a polar solvent. The water-soluble polymer and the counterpart may be the thickener according to the first embodiment. Stated differently, the composition of the present disclosure includes the thickener according to the first embodiment, and a polar solvent. In the description below, "thickener" basically refers to the thickener according to the first embodiment.

### {Thickener}

The above explanation on the water-soluble polymer and the counterpart of the thickener is incorporated herein, and explanation thereon is omitted below.

The content by percentage of the thickener relative to the mass of the composition is preferably 0.1% by mass or greater, more preferably 0.5% by mass or greater, even more preferably 1% by mass or greater, even more preferably 1.5% by mass or greater. If the thickener is less than 0.1% by mass, a sufficient thickening effect cannot be obtained. The content by percentage of the thickener relative to the mass of the composition may be 15% by mass or less. Depending on the desired viscosity, the content by percentage of the thickener relative to the mass of the composition may be 10% by mass or less, 8% by mass or less, 6% by mass or less, or 5% by mass or less.

### {Polar Solvent}

The polar solvent is preferably water and/or a non-aqueous system solvent (organic polar solvent) capable of dissolving the aforementioned water-soluble polymer.

"Non-aqueous system solvents (non-aqueous solvents)" refer to organic solvents other than nonpolar (apolar) solvents such as hydrocarbon oils. It is preferred that the organic polar solvent includes, for example, a functional group capable of creating polarity in a molecule-for example, includes a functional group such as a hydroxy group (OH group), a polyoxyalkylene chain (e.g., a polyoxyethylene chain, a polyoxypropylene chain, etc.), or a phenyl group, or has an asymmetric structure. Preferably, the non-aqueous polar solvent is hydrophilic-i.e., has mutual solubility with water.

Examples of non-aqueous polar solvents that may be used may include the following solvents.

Examples of water-soluble alcohols may include at least one type selected from lower alcohols, polyhydric alcohols, polyhydric alcohol polymers, dihydric alcohol alkyl ethers, dihydric alcohol alkyl ethers, dihydric alcohol ether esters, glycerin monoalkyl ethers, sugar alcohols, monosaccharides, oligosaccharides, polysaccharides, and derivatives of the above.

Examples of the lower alcohol may include ethanol, propanol, isopropanol, isobutyl alcohol, t-butyl alcohol, and the like.

Examples of the polyhydric alcohol may include dihydric alcohol (such as ethylene glycol, propylen glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, octylene glycol, etc); trihydric alcohol (such as glycerin, trimethylolpropane, etc); tetrahydric alcohol (such as such as pentaerythritol such as 1,2,6-hexanetriol, etc); pentahydric alcohol (such as xylitol, etc); hexahydric alcohol (such as sorbitol, mannitol, etc); polyhydric alcohol polymer (such as diethylene glycol, dipropylene glycol, triethylene glycol, polypropylene glycol, tetraethylene glycol, diglycerin, polyethylene glycol, triglycerin, tetraglycerin, polyglycerin, etc); dihydric alcohol alkyl ethers (such as ethylene glycol monomethyl ether, ethylene glycol monoethyl ether, ethylene glycol monobutyl ether, ethylene glycol monomphenyl ether, ethylene glycol monohexyl ether, ethylene glycol mono2-methylhexyl ether, ethylene glycol isoamyl ether, ethylene glycol benzil ether, ethylene glycol isopropyl ether, ethylene glycol dimethyl ether, ethylene glycol diethyl ether, ethylene glycol dibutyl ether, etc); dihydric alcohol alkyl ethers (such as diethylene glycol monomethyl ether, diethylene glycol monoethyl ether, diethylene glycol monombutyl ether, diethylene glycol dimethyl ether, diethylene glycol diethyl ether, diethylene glycol butyl ether, diethylene glycol methylethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether, etc); dihydric alcohol ether ethers (such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disaccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate, etc); glycerin monoalkyl ether (such as chimyl alcohol, selachyl alcohol, batyl alcohol, etc); sugar alcohol (such as sorbitol, maltitol, maltotriose, mannitol, sucrose, erythritol, glucose, fructose, starch sugar, maltose, xylitol, starch sugar hydrogenated alcohol, etc); glycolide, tetrahydrofurfuryl alcohol; POE-tetrahydrofurfuryl alcohol; POP/POE-butyl ether; tripolyoxypropylene glycerin ether; POP-glycerin ether; POP-glycerin ether phosphoric acid; POP/POE-pentaerythritol ether; polyglycerin, and the like.

Examples of the monosaccharides may include at least one selected from triose (such as D-glyceryl aldehyde, dihydroxyacetone, etc); tetrose (such as D-erythrose, D-erythrulose, D-threose, erythritol, etc); pentaose (such as L-arabinose, D-xylose, L-lyxose, D-arabinose, D-ribose, D-ribulose, D-xylulose, L-xylulose, etc); hexalose (such as D-glucose, D-talose, D-psicose, D-galactose, D-fructose, L-galactose, L-mannose, D-tagatose, etc); heptose (such as aldoheptose, heptulose, etc); octose (such as octulose, etc); deoxy sugar (such as 2-deoxy-D-ribose, 6-deoxy-L-galactose, 6-deoxy-L-mannose, etc); amino sugar (such as D-glucosamine, D-galactosamine, sialic acid, amino uronic acid, muramic acid, etc); uronic acid (such as D-grucuronic acid, D-mannuronic acid, L-guluronic acid, D-garacturonic acid, L-iduronic acid, etc) and the like.

Examples of the oligosaccharide may include at least one selected from sucrose, guntianose, umbelliferose, lactose, planteose, isolignoses, α,α-trehalose, raffinose, lignoses, umbilicin, stachyose, verbascoses, and the like.

Examples of the polysaccharide may include at least one selected from cellulose, quince seed, chondroitinsulfate, starch, galactan, dermatan sulfate, glycogen, acasia gum, heparansulfate, hyaluronan, gum tragacanth, keratan sulfate, chondoroitin, xanthan gum, mucoitin sulfate, guar gum, dextran, keratosulfate, locust bean gum, succinoglycan, caronic acid, and the like.

Examples of other polyols may include at least one polyol selected from polyoxyethylene methyl glucoside (Glucam E-10), polyoxypropylene methyl glucoside (Glucam P-10), and the like.

Examples of other organic solvents may include acetic acid, formic acid, acetone, dimethylsulfoxide, acetonitrile, tetrahydrofuran, and N,N-dimethylformamide.

Among the aforementioned polar solvents, it is possible to suitably use at least one selected from, for example, dipropylene glycol, 1,3-butylene glycol, glycerin, polyethylene glycol, polypropylene glycol, octyl methoxycinnamate, octocrylene , homosalate , and octyl salicylate.

The content by percentage of the polar solvent relative to the mass of the composition is preferably 20% by mass or greater. If the content of the polar solvent is less than 20% by mass, it is not possible to ensure a viscosity that satisfies desired handleability, and also, the amount of addition of oily solvents cannot be increased. The content by percentage of the polar solvent can be determined depending on the viscosity of the composition to be obtained. In cases where it is desired to increase the viscosity of the composition, the content by percentage of the polar solvent is preferably 25% by mass or greater, more preferably 30% by mass or greater, even more preferably 35% by mass or greater, even more preferably 40% by mass or greater, further more preferably 45% by mass or greater. The upper limit of the content by percentage of the polar solvent can be determined as appropriate depending on the purpose of the composition. For example, the content by percentage of the polar solvent to the mass of the composition may be 98% by mass or less, 95% by mass or less, 90% by mass or less, 85% by mass or less, 80% by mass or less, 75% by mass or less, 70% by mass or less, 65% by mass or less, 60% by mass or less, 55% by mass or less, or 50% by mass or less.

The polar solvent may include water. Water may originally be included in the polar solvent etc. For the water, it is possible to use any type of water used for cosmetics, quasi-pharmaceutical products, etc., with usable examples including purified water, ion-exchanged water, and tap water. By including a small amount of water in the composition, the thickening effect and transparency can be improved. The content by percentage of water in the polar solvent is preferably 0.5% by mass or greater, more preferably 1% by mass or greater, even more preferably 2% by mass or greater.

If the amount of water to the amount of the polar solvent is too large, emulsification may occur, which may deteriorate transparency, and also, separation of water may occur. From the viewpoint of transparency, the content by percentage of water in the polar solvent is preferably 15% by mass or less, more preferably 12% by mass or less, more preferably 10% by mass or less. The content by percentage of water in the polar solvent may be, for example, 8% by mass or less, 5% by mass or less, 3% by mass or less, 2% by mass or less, or 1% by mass or less. The content by percentage of water in the thickened composition relative to the mass of the composition is preferably 5% by mass or less. In cases where it is desired to increase viscosity while maintaining transparency, it is preferred to set the content by percentage of water to a high value within the aforementioned range.

### {Oily Component}

The composition of the present disclosure may further include an oily component. Preferably, the oily component can dissolve the polar solvent, and/or is soluble in the polar solvent.

Examples of the oily component that may be used include liquid oils, solid fats, waxes, hydrocarbons, higher fatty acids, higher alcohols, synthetic ester oils, and silicone oils.

Examples of the liquid oil that may be used may include avocado oil, camellia oil, turtle oil, macadamia nut oil, corn oil, mink oil, olive oil, rapeseed oil, egg yolk oil, sesame oil, par chic oil, wheat germ oil, southern piece oil, castor oil, linseed oil, safflower oil, cotton seed oil, perilla oil, soybean oil, groundnut oil, brown real oil, torreya oil, rice bran oil, Chinese tung oil, Japanese tung oil, jojoba oil, germ oil, triglycerol, and the like.

Examples of the solid fat that may be used may include cacao butter, coconut oil, horse fat, hydrogenated coconut oil, palm oil, beef tallow, sheep tallow, hydrogenated beef tallow, palm kernel oil, lard, beef bones fat, Japan wax kernel oil, hardened oil, hoof oil, Japan wax, hydrogenated caster oil, and the like.

Examples of the waxes that may be used may include beeswax, candelilla wax, cotton wax, carnauba wax, bayberry wax, insect wax, spermaceti, montan wax, bran wax, lanolin, kapok wax, lanolin acetate, liquid lanolin, sugarcane wax, lanolin fatty acid isopropyl ester, hexyl laurate, reduced lanolin, jojoba wax, hardened lanolin, shellac wax, POE lanolin alcohol ether, POE lanolin alcohol acetate, POE cholesterol ether, lanolin fatty acid polyethylene glycol, POE hydrogenated lanolin alcohol ether, and the like.

Examples of the hydrocarbon oils that may be used may include liquid paraffin, ozocerite, squalane, pristane, paraffin, ceresin. squalene, vaseline, microcrystalline wax, isododecane, isohexadecane, and the like.

Examples of the higher fatty acid that may be used may include lauric acid, myristic acid, palmitic acid, stearic acid, behenic acid, oleic acid, undecylenic acid, tallic acid, isostearic acid, linoleic acid, linolenic acid, eicosapentaenoic acid(EPA), docosahexaenoic acid(DHA) and the like.

Examples of the higher alcohol that may be used may include linear alcohol (such as lauryl alcohol, cetyl alcohol, stearyl alcohol, behenyl alcohol, myristyl alcohol, oleyl alcohol, and cetostearyl alcohol); branched-chain alcohol (such as monostearylglycerin ether (batyl alcohol), 2-decyltetradecinol, lanolin alcohol, cholesterol, phytosterol, hexyldodecanol, isostearyl alcohol, and octyldodecanol) and the like.

Examples of the synthesis ester oils that may be used may include isopropyl myristate, cetyl octanoate, octyldodecyl myristate, isopropyl palmitate, butyl stearate, hexyl laurate, myristyl myristate, decyl oleate, hexyldecyl dimethyl octanoate, cetyl lactate, myristyl lactate, lanolin acetate, isocetyl stearate, isocetyl isostearate, cholesteryl 12-hydroxy stearate, ethylene glycol di-2-ethyl hexanoate, di-penta erythritol fatty acid ester, N-alkyl glycol monoisostearate, neopentyl glycol dicaprate, diisostearyl malate, glyceryl di-2-heptyl undecanoate, trimethyrol propane tri-2-ethyl hexanoate, trimethyrol propane triisostearate, pentaerythritol tetra-2-ethyl hexanoate, glyceryl tri-2-ethyl hexanoate, glyceryl trioctanoate, glyceryl triisopalmitate, trimethyrol propane triisostearate, cetyl 2-ethylhexanoate, 2-ethylhexyl palmitate, glyceryl trimyristate, glyceride tri-2-heptyl undecanoate, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, triethyl citrate, and the like.

Examples of the silicone oil may include silicone compounds such as dimethylpolysiloxane, methylhydrogenpolysiloxane, methylphenylpolysiloxane, stearoxymethylpolysiloxane, polyether-modified organopolysiloxane, fluoroalkyl/polyoxyalkylene co-modified organopolysiloxane, alkyl-modified organopolysiloxane, terminal-modified organopolysiloxane, fluorine-modified organopolysiloxane, amino-modified organopolysiloxane, silicone gel, acrylic silicone, trimethylsiloxysilicic acid, silicone RTV rubber and the like.

Among the aforementioned oily components, it is preferred that the oily component is at least one selected from hydrocarbon oils, ester oils, and silicone oils.

### {Oil-Soluble Active Component}

The composition of the present disclosure may further include, as an oily component, an oil-soluble active component such as a UV absorber. Preferably, the oil-soluble active component is soluble in a solvent of the oily component. The composition may include a water-soluble active component such as a water-soluble UV absorber.

Examples of the oil-soluble ultraviolet light absorbers may include benzoic acid family ultraviolet light absorber (such as p-aminobenzoic acid (hereinafter abbreviated as PABA), PABA monoglycerine ester, N,N-dipropoxy PABA ethyl ester, N,N-diethoxy PABA ethyl ester, N,N-dimethyl PABA ethyl ester, N,N-dimethyl PABA butyl ester, etc); anthranilic acid family ultraviolet light absorber (such as homomenthyl N-acetylanthranilate etc); salicylic acid family ultraviolet light absorber (such as amyl salicylate, menthyl salicylate, homomenthyl salicylate, octyl salicylate, phenyl salicylate, benzyl salicylate, p-isopropanolphenyl salicylate, etc); cinnamic acid family ultraviolet light absorber (such as octyl methoxycinnamate, ethyl 4-isopropylcinnamate, methyl 2,5-diisopropylcinnamate, ethyl 2,4-diisopropylcinnamate, methyl 2,4-diisopropylcinnamate, propyl p-methoxycinnamate, isopropyl p-methoxycinnamate, isoamyl p-methoxycinnamate, octyl p-methoxycinnamate (2-ethylhexyl p-methoxycinnamate), 2-ethoxyethyl p-methoxycinnamate, cyclohexyl p-methoxycinnamate, ethyl α-cyano-β-phenylcinnamate, 2-ethylhexyl α-cyano-β-phenylcinnamate, glyceryl mono-2-ethylhexanoyl-diparamethoxy cinnamate, 3-methyl-4-[methylbis(trimethylsiloxy)silyl]butyl 3,4,5-trimethoxycinnamate, etc); 2-phenyl-5-methylbenzoxazol; 2,2'-hydroxy-5-methylphenylbenzotriazol, 2-(2'-hydroxy-5'-t-octylphenyl) benzotriazol, 2-(2'-hydroxy-5'-methylphenylbenzotriazol; dibenzalazine; dianisoylmethane; 4-methoxy-4'-t-butyldibenzoylmethane; 5-(3,3-dimethyl-2-norbornylidene)-3-pentane-2-one; octocrylene; and the like.

Examples of the water-soluble ultraviolet light absorbers may include benzophenone family ultraviolet light absorber (such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, 4-hydroxy-3-carboxybenzophenone, etc); benzimidazole family ultraviolet light absorber (such as phenylbenzimidazole-5-sulfonic acid and salt thereof, phenylene-bis-benzimidazole-tetrasulfonic acid and salt thereof, etc.); 3-(4'-methylbenzylidene)-d,l-camphor and 3-benzylidene-d,l-camphor; urocanic acid, ethyl urocanate, and the like.

The content by percentage of the oily component relative to the mass of the composition is preferably 85% by mass or less, more preferably 70% by mass or less, even more preferably 60% by mass or less. If the content of the oily component exceeds 85% by mass, transparency will deteriorate due to emulsification etc., and also a feeling of stickiness will increase. The lower limit of the content by percentage of the oily component can be determined as appropriate depending on the purpose of the composition. The content by percentage of the oily component relative to the mass of the composition may be, for example, 10% by mass or greater, 20% by mass or greater, 30% by mass or greater, or 40% by mass or greater.

The composition of the present disclosure may include, as appropriate and as necessary, other components-such as other thickeners, anionic surfactants, cationic surfactants, amphoteric surfactants, nonionic surfactants, powders, water-soluble polymers, film-forming agents, metal ion sequestering agents, amino acids, organic amines, polymer emulsions, pH adjusters, skin nutrients, vitamins, antioxidants, antioxidant aids, and perfumes-in amounts that do not inhibit the effects of the present disclosure.

Examples of other component that may be admixed are listed below. At least one of the following components may be added to the composition of the present disclosure.

Examples of other thickeners may include gum arabic, carrageenan, karaya gum, tragacanth gum, carob gum, quince seed (marmelo), casein, dextrin, gelatin, sodium pectate, sodium alginate, methyl cellulose, ethyl cellulose, carboxymethyl cellulose (CMC), hydroxyethyl cellulose, hydroxypropyl cellulose, polyvinyl alcohol (PVA), polyvinylmethyl ether (PVM), PVP (polyvinyl pyrrolidone), polysodium acrylate, carboxyvinyl polymer, locust bean gum, guar gum, tamarind gum, dialkyldimethylammonium sulfate cellulose, xanthan gum, aluminum magnesium silicate, bentonite, hectorite, aluminum magnesium silicate (Veegum), sodium magnesium silicate (Laponite), silicic acid anhydride gellan gum, and Tremella fuciformis polysaccharide.

Examples of the anionic surfactants that may be used may include fatty acid soap (such as sodium laurate, and sodium palmitate); higher alkyl sulfate ester salt (such as sodium lauryl sulfate, and potassium lauryl sulfate); alkyl ether sulfate ester salt (such as POE-lauryl sulfate triethanolamine, and sodium POE-lauryl sulfate); N-acyl sarcosinic acid (such as sodium lauroyl sarcocinate); higher fatty acid amide sulfonate (such as sodium N-stearoyl-N-methyltaurate, sodium N-myristoyl-N-methyltaurate, sodium methyl cocoyl taurate, and sodium laurylmethyl taurate); phosphate ester salt (sodium POE-oleylether phosphate, POE-stearylether phosphate, potassium cetyl phosphate); sulfosuccinate (such as sodium di-2-ethylhexyl sulfosuccinate, sodium monolauroyl monoethanolamide polyethylene sulfosuccinate, and sodium lauryl polypropylene glycol sulfosuccinate); alkylbenzene sulfonate (such as sodium linear dodecylbenzene sulfonate, triethanolamine linear dodeylbenzene sulfonate, and linear dodecylbenzene sulfonate); higher fatty acid ester sulfate ester salt (such as sodium hydrogenated gryceryl cocoate sulfate); N-acyl glutamate (such as monosodium N-lauroyl glutamate, disodium N-stearoyl glutamate, and monosodium N-myristoyl-L-glutamate); sulfonated oil (such as Turkey red oil); POE-alkyl ether carboxylic acid; POE-alkyl aryl ether carboxylate; α-olefine sulfonate; higher fatty acid ester sulfonate; secondary alcohol sulfate ester salt; higher fatty acid alkylolamide sulfate ester salt; sodium lauroyl monoethanolamide succinate; N-palmitoyl asparaginate ditriethanolamine; sodium casein; and the like.

Examples of the cationic surfactants may include alkyltrimethyl ammonium salt (such as stearyltrimethyl ammonium chloride, lauryltrimethyl ammonium chloride); alkylpyridinium salt (such as cetylpyridinium chloride); dialkyldimethyl ammonium salt (such as distearyldimethyl ammonium chloride); poly (N,N'-dimethyl-3,5-methylenepiperidinium) chloride; alkyl quaternary ammonium salt; alkyldimethylbenzyl ammonium salt; alkylisoquinolinium salt; dialkylmorphonium salt; POE alkylamine; alkylamine salt; polyamine fatty acid derivative; amyl alcohol fatty acid derivative; benzalkonium chloride; benzethonium chloride, and the like.

Examples of the amphoteric surfactant that may be used may include: imidazoline-based amphoteric surfactant (such as sodium 2-undecyl-N,N,N-(hydroxyethylcarboxymethyl)-2-imidazoline and 2-cocoyl-2-imidazolinium hydroxide-1-carboxyethyloxy disodium salt); and betaine-based surfactant (such as 2-heptadecyl-N-carboxymethyl-N-hydroxyethyl imidazolinium betaine, lauryl dimethylaminoacetic acid betaine, alkyl betaine, amidobetaine, and sulfobetaine).

Examples of the lipophilic nonionic surfactants may include sorbitan fatty acid ester (such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2 ethylhexylate, diglycerol sorbitan tetra-2 ethylhexylate, etc); glyceryl polyglyceryl fatty acid (such as glyceryl monocotton oil fatty acid, glyceryl monoerucate, glyceryl sesquioleate, glyceryl monostearate, glyceryl α, α'-oleate pyroglutamate, glyceryl monostearate malate, etc); propylene glycol fatty acid ester (such as propylene glycol monostearate, etc); hydrogenated caster oil derivative; glyceryl alkyl ether, and the like.

Examples of the hydrophilic nonionic surfactants that may be used may include POE sorbitan fatty acid ester (such as POE sorbitan monooleate, POE sorbitan monostearate, POE sorbitan monooleate, POE sorbitan tetraoleate); POE sorbit fatty acid ester (such as POE sorbit monolaurate, POE sorbit monooleate, POE sorbit pentaoleate, POE sorbit monostearate), POE glyceryl fatty acid ester (such as POE monooleate such as POE glyceryl monostearate, POE glyceryl monoisostearate, POE glyceryl triisostearate); POE fatty acid ester (such as POE distearate, POE monodioleate, ethyleneglycol distearate); POE alkyl ether (such as POE lauryl ether, POE oleyl ether, POE stearyl ether, POE behenyl ether, POE-2-octyldodecyl ether, POE cholestanol ether); puluronic type (such as Puluronic), POE/POP alkyl ethers (such as POE/POP cetyl ether, POE/POP 2-decyltetradecyl ether, POE/POP monobutyl ether, POE/POP hydrogenated lanoline, POE/POP glycerin ether); tetra POE/tetra POP ethylenediamine condensation products (such as Tetronic); POE castor oil hydrogenated castor oil derivative (such as POE caster oil, POE hydrogenated caster oil, POE hydrogenated caster oil monoisostearate, POE hydrogenated castor oil triisostearate, POE hydrogenated caster oil monopyroglutamate monoisostearate diester, POE hydrogenated oil maleate); POE beeswax/lanoline derivative (such as POE sorbitol beeswax); alkanolamide (such as coconut oil fatty acid diethanolamide, lauric acid monoethanolamide, fatty acid isopropanolamide); POE propyleneglycol fatty acid ester; POE alkyl amines; POE fatty acid amide; sucrose fatty acid ester; alkylethoxydimethylamine oxide; trioleyl phosphoric acid and the like.

Examples of the powder bodies may include inorganic powder (such as talc, kaolin, mica, sericite, muscovite, phlogopite, synthetic mica, lepidolite, biotite, vermiculite, magnesium carbonate, calcium carbonate, aluminum silicate, barium silicate, calcium silicate, magnesium silicate, strontium silicate, tungstate, magnesium, silica, zeolite, glass, barium sulfate, calcined calcium sulfate (calcined gypsum), calcium phosphate, fluorine apatite, hydroxyapatite, ceramic powder, metallic soap (such as zinc myristate, calcium palimitate, and aluminum stearate), and boron nitride, etc); organic powder (such as polyamide resin powder (nylon powder), polyethylene powder, polymethylmethacrylate powder, polystyrene powder, styrene-acrylic acid copolymer powder, benzoguanamine resin powder, poly(tetrafluroethylene) powder, and cellulose powder, silicone resin powder, silk powder, wool powder, urethane powder, etc); inorganic white family pigment (such as titanium dioxide, zinc oxide, etc); inorganic red family pigment (such as iron oxide (colcothar), iron titanate, etc); inorganic brown family pigment (such as γ-iron oxide, etc); inorganic yellow family pigment (such as yellow iron oxide, loess, etc); inorganic black family pigment (such as black iron oxide, carbon black, lower titanium oxide, etc); inorganic purple family pigment (such as manganese violet, cobalt violet, etc); inorganic green family pigment (such as chrome oxide, chrome hydroxide, cobalt titanate, etc); inorganic blue family pigment (such as ultramarine, iron blue, etc); pearl pigment (such as titanium oxide coated mica, titanium oxide coated bismuth oxychloride, titanium oxide coated talc, colored titanium oxide coated mica, bismuth oxychloride, argentine, etc); metal powder pigment (such as aluminum powder, copper powder, etc); organic pigment such as zirconium, barium, or aluminum lake (such as organic pigment such as Red No.201, Red No.202, Red No.204, Red No.205, Red No.220, Red No.226, Red No.228, Red No.405, Red No.201, Orange No.203, Orange No.204, Yellow No.205, Yellow No.401, Blue No.401, Red No.3, Red No.104, Red No.106, Red No.227, Red No.230, Red No.401, Red No.505, Orange No.205, Yellow No.4, Yellow No.5, Yellow No.202, Yellow No.203, Green No.3, and Blue No.1, etc); natural pigment (such as chlorophyll, β-carotene, etc) and the like.

The content by percentage of the powder relative to the mass of the composition is preferably 5% by mass or less, more preferably 3% by mass or less. If more than 5% by mass of powder is included, the composition may leave a white cast on the skin when applied to the skin as a cosmetic, or may deteriorate the transparency of the composition.

Examples of the moisturizers may include polyethylene glycol, propylene glycol, glycerin, 1,3-butylene glycol, xylitol, sorbitol, maltitol, chondroitin sulfate, hyaluronic acid, mucoitin sulfate, charonic acid, atelocollagen, cholesteryl 12-hydroxystearate, sodium lactate, bile salt, dl-pyrrolidone carboxylate, alkyleneoxide derivative, short-chain soluble collagen, diglycerin (EO)PO adduct, chestnut rose extract, yarrow extract, melilot extract, and the like.

Examples of the natural water-soluble polymer may include plant-based polymer (such as gum Arabic, gum tragacanth, galactan, guar gum, locust bean gum, gum karaya, carrageenan, pectine, agar, quince seed (cydonia oblonga), algae colloid (brown algae extract), starch (rice, corn, potato, wheat) ,glicyrrhizic acid); microorganism based polymer (such as xanthan gum, dextran, succinoglycan, pullulan, etc), animal-based polymer (such as collagen, casein, albumin, gelatine, etc) and the like.

Examples of the semisynthetic water-soluble polymer may include starch-based polymer (such as carboxymethyl starch, methylhydroxypropyl starch, etc); cellulose-based polymer (such as methylcellulose, ethylcellulose, methylhydroxypropylcellulose, hydroxyethylcellulose, cellulose sodium sulfate, hydroxypropylcellulose, carboxymethylcellulose, sodium calboxymethyl cellulose, crystalline cellulose, cellulose powder, etc); algin acid-based polymer (such as sodium alginate, propylene glycol alginate ester, etc), and the like.

Examples of the synthetic water-soluble polymer may include vinyl based polymer (such as polyvinyl alcohol, polyvinyl methyl ether, polyvinylpyrrolidone, carboxyvinylpolymer, etc); polyoxyethylene based polymer (such as polyoxyethylenepolyoxypropylene copolymer such as polyethylene glycol 20,000, 40,000 and 60,000, etc); acrylic polymer (such as sodium polyacrylate, polyethylacrylate, polyacrylamide, etc); polyethyleneimine; cationic polymer; and the like.

Examples of the film-forming agent may include an anionic film-forming agent (such as (meta)acrylic acid/(meta)acrylic acid ester copolymer, methyl vinyl ether/maleic anhydride coplymer, etc), a cationic film-forming agent (such as cationic cellulose, diallyldimethylammonium chloride polymer, diallyldimethylammonium chloride/acrylic amide copolymer, etc), a nonionc film-forming agent (such as polyvinyl alcohol, polyvinylpyrrolidone, polyvinyl acetate, polyacrylic ester copolymer, (meta)acrylamide, polymeric silicone, silicone resin, trimethylsiloxysilicate, etc), and the like.

Examples of the metal ion sequestrant may include 1-hydroxyethane-1, 1-diphosphonic acid, 1-hydroxyethane, 1-diphosphonic acid 4Na salt, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid, trisodium hydroxyethyl ethylenediamine triacetate, and the like.

Examples of the amino acid may include neutral amino acid (such as threonine, cysteine, etc); basic amino acid (such as hydroxylysine, etc) and the like. Examples of the amino acid derivative may include sodium acyl sarcosinate (sodium lauroyl sarcosinate), acyl glutamate, sodium acyl β-alanine, glutathione, pyrrolidone carboxylate, and the like.

Examples of the organic amine may include monoethanolamine, diethanolamine, triethanolamine, morpholine, triisopropanolamine, 2-amino-2-methyl-1,3-propanediol, 2-amino-2-methyl-1-propanol, and the like.

Examples of the polymer emulsion may include acrylic resin emulsion, ethyl polyacrylate emulsion, solution of acrylic resin, polyacrylalkylester emulsion, polyvinyl acetate resin emulsion, natural rubber latex, and the like.

Examples of the pH modifier may include buffer such as lactic acid-sodium lactate, citric acid-sodium citrate, succinic acid-sodium succinate, and the like.

Examples of the vitamins may include vitamine A, B1, B2, B6, C, E and derivatives thereof, pantothenic acid and derivatives thereof, biotin, and the like.

Examples of the anti-oxidant may include tocopherols, dibutyl hydroxy toluene, butyl hydroxy anisole, and gallic acid esters, and the like.

Examples of the anti-oxidant aid may include phosphoric acid, citric acid, ascorbic acid, maleic acid, malonic acid, succinic acid, fumaric acid, cephalin, hexamethaphosphate, phytic acid, ethylenediaminetetraacetic acid, and the like.

Examples of other containable compositions may include an antiseptic agent (such as ethylparaben, butylparaben, chlorphenesin, 2-phenoxyethanol, etc); antiphlogistic (such as glycyrrhizinic acid derivatives, glycyrrhetic acid derivatives, salicylic acid derivatives, hinokitiol, zinc oxide, allantoin, etc); a skin-whitening agent (such as placental extract, saxifrage extract, arbutin, etc); various extracts (such as phellodendron bark (cork tree bark), coptis rhizome, lithospermum, peony, swertia herb, birch, sage, loquat, carrot, aloe, mallow, iris, grape, coix seed, sponge gourd, lily, saffron, cnidium rhizome, ginger, hypericum, restharrow, garlic, red pepper, citrus unshiu, Japanese angelica, seaweed, etc); an activator (such as royal jelly, photosenstizer, cholesterol derivatives, etc); a blood circulation promotion agent (such as nonylic acid vanillylamide, nicotine acid benzyl ester, nicotine acid β-butoxyethyl ester, capsaicin, zingerone, cantharides tincture, ichthammol, tannic acid, α-borneol, tocopheryl nicotinate, meso-inositol hexanicotinate, cyclandelate, cinnarizine, tolazoline, acetylcholine, verapamil, cepharanthine, γ-oryzanol, etc); an antiseborrheric agent, (such as sulfur, thianthl, etc); an anti-inflammatory agent (such as tranexamic acid, thiotaurine, hypotaurine, etc), and the like.

The composition of the present disclosure further may inculde, as necessary, caffeine, tannin, verapamil, tranexamic acid and derivatives thereof; various crude drug extracts such as licorice, Chinese quince, Pyrola japonica and the like; drugs such as tocopherol acetate, glycyrrhetinic acid, glycyrrhizic acid and derivatives thereof, or salts thereof; skin-whitening agents such as vitamin C, magnesium ascorbyl phosphate, ascorbic acid glucoside, arbutin, kojic acid and the like; amino acids such as arginine and lysine and the like and derivatives thereof.

The viscosity of the composition of the present disclosure can be set as appropriate depending on the purpose. The viscosity of the composition of the present disclosure is preferably 300 mPa·s or greater, more preferably 500 mPa·s or greater, even more preferably 1,000 mP·as or greater, even more preferably 2,000 mPa·s or greater. If the viscosity is less than 300 mPa·s, the composition becomes likely to drip down, thus deteriorating usability. The viscosity of the composition of the present disclosure is preferably 500,000 mPa·s or less, more preferably 300,000 mPa·s or less, even more preferably 100,000 mPa·s or less, even more preferably 50,000 mPa·s or less, even more preferably 20,000 mPa·s or less. If the viscosity exceeds 500,000 mPa·s, the composition becomes difficult to spread. The viscosity can be measured with a Brookfield viscometer (spindle No. 7; rotation speed: 10 rpm) at 30°C.

As for the transparency of the composition of the present disclosure, it is preferred that the L* value in Lab color space is preferably 90 or greater, more preferably 95 or greater. The Lab color space can be measured with a color difference meter (e.g., COLOR-EYE 7000A (from Gretag Macbeth)).

The composition of the present disclosure can achieve high viscosity and transparency not only in aqueous systems, but also in non-aqueous systems. Thus, the composition of the present disclosure is applicable to products, such as cosmetic products, having high transparency and excellent usability, even when including an oil-soluble active component such as a UV absorber.

The composition of the present disclosure can be manufactured according to known methods, without being limited to a specific method. For example, the thickener of the present disclosure can be manufactured by mixing the aforementioned components.

The composition and the thickener of the present disclosure are described below by way of examples. The composition and the thickener of the present disclosure, however, are not limited to the following working examples. The unit employed for indicating the contents by percentage shown in the Tables is percent by mass (mass%).

### Examples

### {Test Examples 1 to 8}

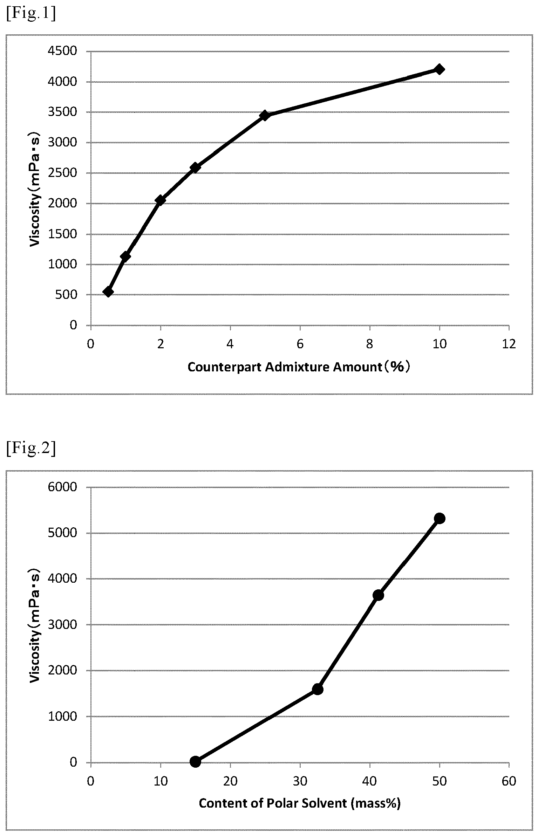

The thickening effect of thickened compositions of the present disclosure was verified. For the water-soluble polymer, a polymer including a pyrrolidone group as a heterocycle and a dimethylamino group as a first polar group was used. For the counterpart, either isostearic acid or lactic acid, including a carboxy group as a second polar group, was used. The non-aqueous system solvent to be thickened was a mixed solvent of: ethanol as a polar solvent; and diisopropyl sebacate as an oily component. Diisopropyl sebacate has a symmetric structure, and can thus be regarded as a non-polar solvent. The viscosity of each composition was measured. The viscosity was measured with a Brookfield viscometer (spindle No. 7; rotation speed: 10 rpm) at 30°C. Table 1 shows compositions employing isostearic acid, and viscosities thereof. Table 2 shows compositions employing lactic acid, and viscosities thereof. Fig. 1 is a graph showing a relationship between viscosity and the counterpart admixture amount in Test Examples 1 to 6. The evaluation criteria for transparency are as described below.

### {Transparency}

A: No turbidity or aggregation was observed.
B: Slight turbidity was observed.
C: Turbidity was observed.
D: Precipitation or aggregation was observed.

As illustrated in Fig. 1, it was found that, by increasing the admixture amount of isostearic acid, the viscosity of the composition increased logarithmically. It is thus considered that isostearic acid, and not only the water-soluble polymer, also contributes to the thickening of the composition. The thickening effect was also verified in Test Examples 7 and 8 using lactic acid instead of isostearic acid. It is thus considered that the water-soluble polymer and the organic acid interact with one another. For example, it is considered that the dimethylamino group of the water-soluble polymer and the carboxy group of the counterpart interact with one another, such as by neutralization or formation of an ion pair, in the polar solvent or in the slight amount of water included in the polar solvent. It is also considered that the polar solvent has a function of dissolving the water-soluble polymer and the counterpart, which are polar compounds.

All of the compositions according to Test Examples 1 to 8 were transparent and exhibited no turbidity etc.

According to Test Examples 1 to 6 in which the counterpart was a fatty acid, it was found that the thickening effect can be improved when the content of the counterpart, with respect to 1 part by mass of the water-soluble polymer, was at least 0.1 parts by mass or greater, preferably 0.2 parts by mass or greater, more preferably 0.5 parts by mass or greater. Further, according to Test Examples 7 and 8 in which the counterpart was lactic acid, it was found that the thickening effect can be improved when the content of the counterpart, with respect to 1 part by mass of the water-soluble polymer, was at least 0.02 parts by mass or greater, preferably 0.05 parts by mass or greater.

**[Table 1]**

| | | Test Example | 1 | 2 | 3 | 4 | 5 | 6 |
|---|---|---|---|---|---|---|---|---|
| (1) | Water-solubl e polymer | Cationic poly mer^{*1} | 2 | 2 | 2 | 2 | 2 | 2 |
| (2) | Counterpart | Isostearic ac id | 0.5 | 1 | 2 | 3 | 5 | 10 |
| (3) | Polar solve nt | Grade-99% e thanol | 55 | 55 | 55 | 55 | 55 | 55 |
| (4) | Oily compo nent | Diisopropyl s ebacate | Balan ce | Balan ce | Balan ce | Balan ce | Balan ce | Balan ce |
| Total | | | 100 | 100 | 100 | 100 | 100 | 100 |
| Viscosity (mPa·s) | | | 550 | 1130 | 2050 | 2590 | 3440 | 4200 |
| Transparency | | | A | A | A | A | A | A |
| *1: Vinyl pyrrolidone/N,N'-dimethylaminoethyl methacrylate/stearyl acrylate/tripropylene glycol diacrylate copolymer (CG polymer from Osaka Organic Chemical Industry Ltd.) | | | | | | | | |

**[Table 2]**

| | Test Example | | 7 | 8 |
|---|---|---|---|---|
| (1) | Water-soluble polymer | Cationic polymer ^{*1} | 2 | 2 |
| (2) | Counterpart | Lactic acid | 0.1 | 0.6 |
| (3) | Polar solvent | Grade-99% ethanol | 55 | 55 |
| (4) | Oily component | Diisopropyl sebacate | Balance | Balance |
| Total | | | 100 | 100 |
| Viscosity (mPa·s) | | | 3700 | 5890 |
| Transparency | | | A | A |

### {Test Examples 9 to 25}

Solvents capable of being thickened by using the thickeners of the present disclosure were studied. Also, transparency was checked for each composition. The compositions and evaluation results of the respective Test Examples are shown in Tables 3 and 4. "IS acid" is an abbreviation for isostearic acid. The evaluation criteria for thickening effect are as described below. The evaluation criteria for transparency are the same as those for Test Examples 1 to 8.

### {Thickening Effect}

A: Thickening effect was observed.
B: Some thickening effect was observed.
C: No thickening effect was observed.

The thickeners of the present disclosure were basically able to exert a thickening effect on polar solvents, such as alcohols. On the other hand, the thickeners of the present disclosure were not able to exert any thickening effect on non-polar solvents, such as oily solvents. It is thus considered that the thickeners of the present disclosure are useful for thickening polar solvents.

However, as shown by Test Example 25, ion-exchanged water could not be thickened, even though it is a polar solvent. This is considered to be because the solubility of isostearic acid to ion-exchanged water is low. It is thus considered that there may be a need to select a counterpart that is soluble in the solvent. For example, in cases where the counterpart is an organic acid, it is considered that a solvent including a polar organic solvent as the main solvent can suitably be thickened. Note, however, that the solvent may include water, as described further below.

As regards transparency, compositions having an excellent thickening effect were also able to achieve high transparency. In contrast, compositions having a poor thickening effect had low transparency due, for example, to turbidity, aggregation, etc. It is thus considered that there is a correlation between the thickening effect and transparency. The reason that transparency became low is considered to be because the ionic polymer and/or the counterpart could not dissolve into the solvent. For example, when comparing Test Examples 3 and 20, Test Example 3, which included a polar solvent, exhibited good results in terms of both thickening properties and transparency, whereas Test Example 20, which did not include a polar solvent, exhibited poor results in terms of both thickening properties and transparency. It is thus considered that the thickener of the present disclosure acts effectively in the presence of a polar solvent capable of dissolving the water-soluble polymer and the counterpart (or a complex thereof).

When comparing Test Examples 9 and 11, it was possible to make the viscosity of grade-95% ethanol higher than that of grade-99% ethanol. It is thus considered that the water content in grade-95% ethanol had an influence on the thickening effect. It is thus considered that it is preferred that the solvent includes a small amount of water (for example, from 2% by mass to less than 10% by mass relative to the mass of the solvent).

**[Table 3]**

| | Test Example | | 9-25 |
|---|---|---|---|
| (1) | Water-soluble polymer | Cationic polymer ^{*1} | 2 |
| (2) | Counterpart | See Table 4 | 2 |
| (3) | Solvent | See Table 4 | 96 |
| Total | | | 100 |

**[Table 4]**

| | Solvent | | Counte rpart | Transpar ency | Thickening effect (Viscosity (mPa·s)) |
|---|---|---|---|---|---|
| | Type | Polarity | | | |
| Test Exa mple 9 | Grade-95% ethanol | Polar | IS acid | A | A(16000) |
| Test Exa mple 10 | Grade-95% ethanol | Polar | Lactic acid | A | A(21400) |
| Test Exa mple 11 | Grade-99% ethanol | Polar | IS acid | A | A(5810) |
| Test Exa | Glycerin | Polar | Lactic | A | A(317600) |
| mple 12 | | | acid | | |
| Test Exa mple 13 | 1,3-butylene glycol | Polar | Lactic acid | A | A(71400) |
| Test Exa mple 14 | Dipropylene glycol | Polar | IS acid | A | A(23400) |
| Test Exa mple 15 | Dipropylene glycol | Polar | Lactic acid | A | A(39700) |
| Test Exa mple 16 | Polyethylene glycol 400 | Polar | Lactic acid | A | A(37000) |
| Test Exa mple 17 | Ethylhexyl methoxycinnam ate | Polar | IS acid | B | B(560) |
| Test Exa mple 18 | Octocrylene | Polar | IS acid | A | A(10200) |
| Test Exa mple 19 | Propylene glycol laurate | Polar | IS acid | A | A(3610) |
| Test Exa mple 20 | Diisopropyl sebacate | Nonpolar | IS acid | C | C |
| Test Exa mple 21 | Glyceryl tri-2-ethylhexano ate | Nonpolar | IS acid | C | C |
| Test Exa mple 22 | Isododecane | Nonpolar | IS acid | D | C |
| Test Exa mple 23 | Methylphenyl polysiloxane ^{*2} | Nonpolar | IS acid | B | C |
| Test Exa mple 24 | Methyl polysiloxane ^{*3} | Nonpolar | IS acid | D | C |
| Test Exa mple 25 | Ion-exchanged water | Polar | IS acid | D | C |
| *2: Silicone KF56 from Shin-Etsu Silicone | | | | | |
| *3: Silicone KF-96A-6T from Shin-Etsu Silicone | | | | | |

### {Test Examples 26 to 29}

Sun-block cosmetics were prepared by using the thickener of the present disclosure, and their viscosity and transparency were verified. The evaluation criteria for transparency are the same as described above. In Test Examples 26 to 29, the content by percentage of grade-95% ethanol, serving as the polar solvent, was varied. The compositions and evaluation results are shown in Table 5. Fig. 2 is a graph showing a relationship between viscosity and the content by percentage of the polar solvent in Test Examples 26 to 29.

Fig. 2 shows that it was possible to increase viscosity when the content by percentage of alcohol, which is the polar solvent, was increased. As explained in relation to Test Examples 3 and 20, this is thought to be because, in Test Example 26, the water-soluble polymer and the counterpart were not dissolved since the amount of the polar solvent was small, and thus viscosity was not increased. Since there is a proportional relationship between viscosity and the content by percentage of the polar solvent, it is considered that the polar solvent also contributes to the thickening effect. According to Fig. 2, it is considered that a viscosity of 500 mPa·s or higher can be achieved when the content by percentage of the polar solvent is 20% by mass or greater. Further, Test Example 26 not only had low viscosity, but also had a slightly lower transparency than Test Examples 27 to 29. This is thought to be because, in Test Example 26, the water-soluble polymer and the counterpart were not dissolved completely since the amount of the polar solvent was small. It is thus considered that the preferable content by percentage of the polar solvent is preferably 20% by mass or greater, more preferably 25% by mass or greater, even more preferably 30% by mass or greater, even more preferably 40% by mass or greater.

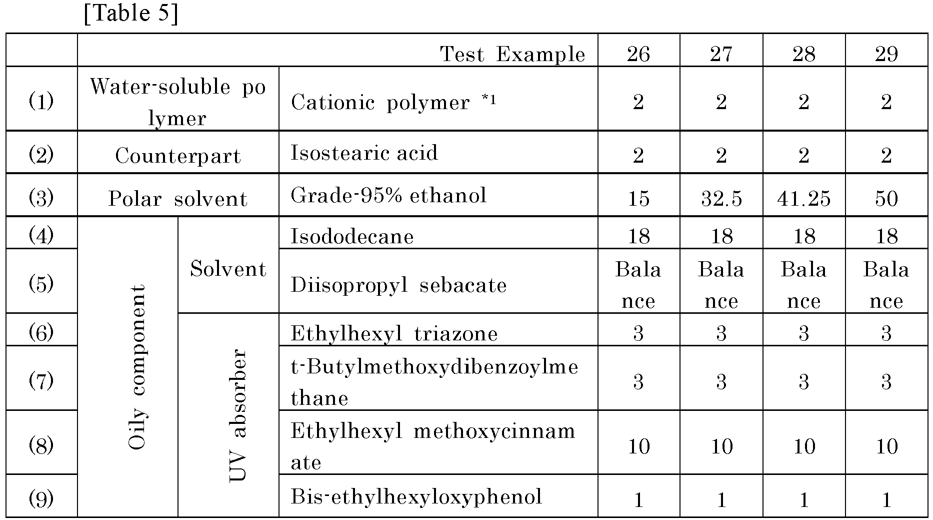

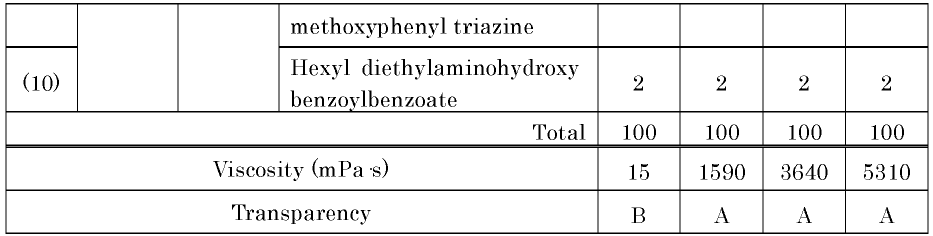

### {Test Examples 30 to 35}

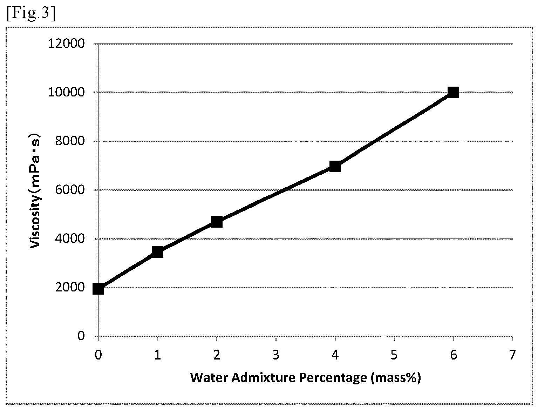

Sun-block cosmetics were prepared by using the thickener of the present disclosure, and their viscosity and transparency were verified. The evaluation criteria for transparency are the same as described above. In Test Examples 30 to 35, grade-99% ethanol was used as the polar solvent, and the admixture percentage of ion-exchanged water was varied. The compositions and evaluation results are shown in Table 6. Fig. 3 is a graph showing a relationship between viscosity and the water admixture percentage in Test Examples 30 to 35.

Even in Test Example 30, which did not include ion-exchanged water, it was possible to observe a thickening effect and achieve sufficient transparency. However, as Fig. 3 shows, viscosity can be proportionally increased by increasing the water admixture amount. It was thus found that the thickener of the present disclosure can achieve thickening, even when containing water. From this result, it is considered that water also contributes to the thickening effect.

In Test Examples 34 and 35, however, emulsification occurred, causing white turbidity. Further, Test Example 35 had a significantly lower viscosity compared to Test Example 34. This is thought to be because, in Test Examples 34 and 35, the increase in the content by percentage of water either lowered the solubility of the thickener (complex between the water-soluble polymer and the counterpart) of the present disclosure, or inhibited the formation of the thickener of the present disclosure. It is thus considered that, in order to improve both thickening properties and transparency, the preferable content by percentage of water in the polar solvent is preferably 10% by mass or less, more preferably 8% by mass or less. It is also considered that the preferable content by percentage of water in the polar solvent is preferably 1% by mass or greater, more preferably 2% by mass or greater.

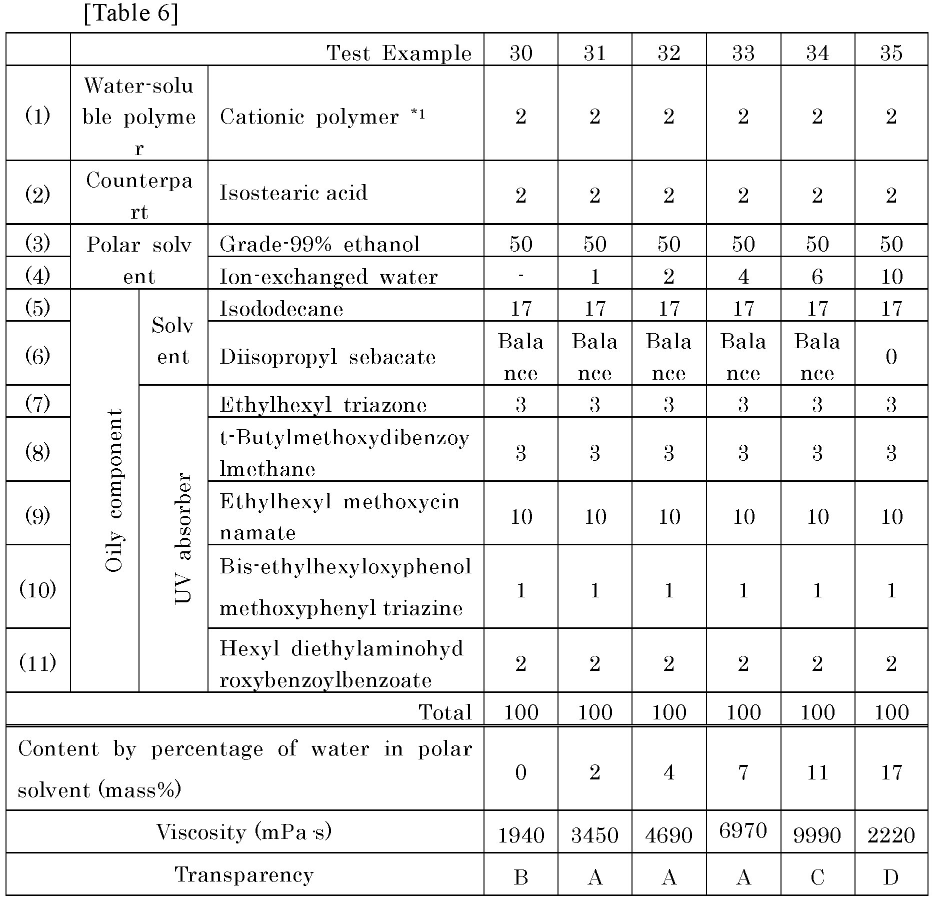

### {Test Examples 36 and 37}

In Test Examples 1 to 35, a cationic/basic polymer was used for the ionic polymer, and anionic/acidic organic compounds were used for the counterpart. In Test Example 36, an anionic/acidic polymer was used for the ionic polymer and a cationic organic compound was used for the counterpart, and whether it was possible to achieve a thickening effect and transparency was verified. In Test Example 37, a carboxyvinyl polymer, which is used as a thickener for water, was used for the ionic polymer. The compositions and evaluation results are shown in Table 7. The evaluation criteria for thickening effect and transparency are the same as those described above.

In Test Example 36, it was verified that a thickening effect was achieved even by the combination of an anionic water-soluble polymer and a cationic counterpart. It was also possible to obtain a composition having high transparency. It was thus possible to achieve a thickening effect and transparency, not only by the combination of a cationic/basic water-soluble polymer and an anionic/acidic counterpart, but also by a combination of an anionic/acidic water-soluble polymer and a cationic counterpart.

The counterpart used in Test Example 37 is a chloride of a quaternary amine, and is thus considered not basic. Thus, in the thickener of the present disclosure, there is a possibility that the water-soluble polymer and the counterpart form an ion pair.

In Test Example 37, viscosity could not be increased, and also, transparency could not be improved. This is considered to be because the carboxyvinyl polymer either did not form a complex with distearyldimonium chloride, or did not dissolve in the polar solvent and oily components due to the lack of a hydrophobic moiety. It is thus considered that a highly hydrophilic thickener typically used as a thickener for water cannot thicken non-aqueous system solvents.

**[Table 7]**

| | Test Example | | | 36 | 37 |
|---|---|---|---|---|---|
| (1) | Water-soluble polymer | | Anionic polymer ^{*4} | 5 | - |
| | | | Anionic polymer ^{*5} | - | 5 |
| (2) | Counterpart | | Distearyldimonium chloride | 3 | 3 |
| (3) | Polar solvent | | Grade-95% ethanol | 50 | 50 |
| (4) | Oily com ponent | Solvent | Diisopropyl sebacate | 27 | 27 |
| (5) | | UV absorber | Ethylhexyl methoxycinnamate | 15 | 15 |
| Total | | | | 100 | 100 |
| Thickening effect | | | | A | C |
| Viscosity (mPa·s) | | | | (9230) | (-) |
| Transparency | | | | A | D |
| *4: Acrylates/C10-30 alkyl acrylate crosspolymer (PEMULEN (registered trademark) from Lubrizol Advanced Materials, Inc.) | | | | | |
| *5^{:} Carboxyvinyl polymer (Carbopol (registered trademark) from Lubrizol Advanced Materials, Inc.) | | | | | |

### {Test Examples 38 to 43}

In Test Examples 38 to 43, the types of counterparts in the thickeners of the present disclosure were varied, and whether it was possible to achieve a thickening effect and transparency was verified. The types of oily components were also varied from those in the aforementioned Examples. The compositions and evaluation results are shown in Table 8.

All of the counterparts listed in Table 8 were capable of achieving a thickening effect and high transparency. It is thus considered that compounds having a polar functional group, such as a carboxy group, are applicable as counterparts in the thickeners of the present disclosure. It was also found that, for example, fatty acids and derivatives thereof are applicable as counterparts. The number of carbon atoms in the counterparts used in Working Examples 38 to 43 was from 16 to 22. The counterparts also had a C₆₋₂₀ carbon chain (straight chain). It was also found that siloxane compounds are applicable as counterparts.

Thickening was possible, even when oily components different from the oily components used in the aforementioned Test Examples were added. It is thus considered that the thickeners of the present disclosure are applicable to various oily components having mutual solubility with polar solvents.

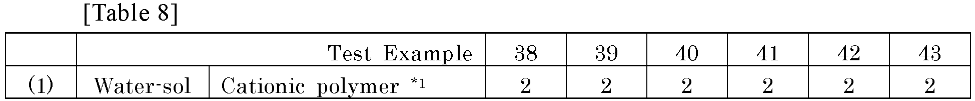

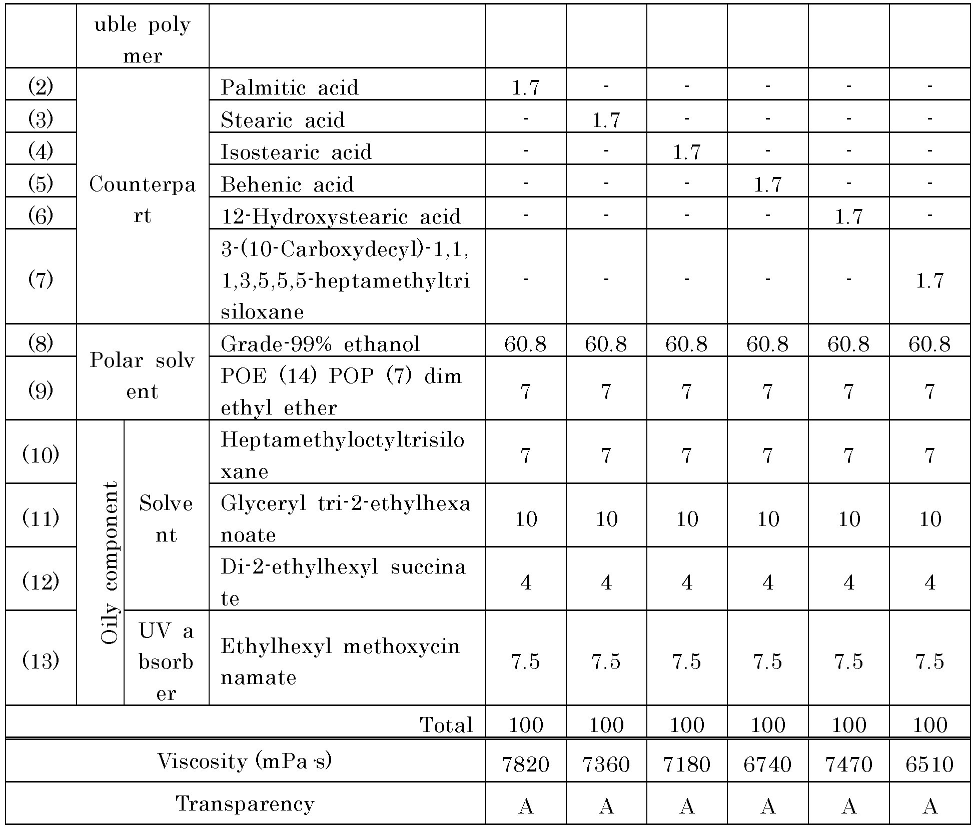

The composition and thickener and manufacturing methods thereof of the present invention have been described according to the foregoing embodiments and examples, but the invention is not limited to the foregoing embodiments and examples and may encompass various transformations, modifications, and improvements made to the various disclosed elements (including elements disclosed in the Claims, Description, and Drawings) within the scope of the invention and according to the fundamental technical idea of the present invention. Further, various combinations, substitutions, and selections of the various disclosed elements are possible within the scope of the claims of the invention.

Further issues, objectives, and embodiments (including modifications) of the present invention are revealed also from the entire disclosure of the invention including the Claims.

The numerical ranges disclosed herein are to be construed in such a manner that arbitrary numerical values and ranges falling within the disclosed ranges are treated as being concretely described herein, even where not specifically stated.

### Industrial Applicability

The composition and thickener of the present disclosure are suitably applicable to cosmetics and external skin preparations used directly with the hands. The composition of the present disclosure is suitably applicable to cosmetics and external skin preparations that are applied to or rubbed into the skin and/or hair.

The composition and thickener of the present disclosure are suitably applicable, for example, to oil-in-water-type products, water-in-oil-type products, products including a main solvent other than water, and products including oil-soluble components as active components. Particularly, the composition and thickener are suitably applicable to products requiring transparency.

## Claims

1. A thickener for a non-aqueous system, the thickener comprising:
a water-soluble polymer; and
a counterpart that ionically and/or electrostatically interacts with said water-soluble polymer.

2. The thickener according to claim 1, wherein said non-aqueous system includes a polar organic solvent as a main solvent.

3. The thickener according to claim 1 or 2, wherein:
said water-soluble polymer includes a first polar group;
said counterpart includes a second polar group; and
one of said first polar group and said second polar group is an anionic functional group, and the other is a cationic functional group.

4. The thickener according to claim 3, wherein said first polar group and said second polar group are each either a carboxy group or an amine.

5. The thickener according to claim 3 or 4, wherein said water-soluble polymer further includes a heterocycle.

6. The thickener according to claim 5, wherein said heterocycle is a lactam.

7. The thickener according to claim 5 or 6, wherein said water-soluble polymer is a copolymer of a first component including said first polar group and a second component including said heterocycle.

8. The thickener according to claim 7, wherein said first component is a 2-(dimethylamino)ethyl methacrylate component, and said second component is a vinylpyrrolidone component.

9. The thickener according to claim 7 or 8, wherein said water-soluble polymer is a copolymer further including a cross-linking third component.

10. The thickener according to any one of claims 7 to 9, wherein said water-soluble polymer is a copolymer further including a fourth component including an alkyl acrylate and/or an acrylamide.

11. The thickener according to claim 10, wherein, in said water-soluble polymer, with respect to the total amount of the first to fourth components:
the proportion of said first component is from 15 to 85% by mass;
the proportion of said second component is from 20 to 80% by mass;
the proportion of said third component is from 0 to 20% by mass; and
the proportion of said fourth component is from 0 to 60% by mass.

12. The thickener according to any one of claims 1 to 11, wherein said water-soluble polymer is a vinyl pyrrolidone/N,N-dimethylaminoethyl methacrylate/stearyl acrylate/tripropylene glycol diacrylate copolymer.

13. The thickener according to any one of claims 1 to 12, wherein said counterpart is an anionic organic compound.

14. The thickener according to any one of claims 1 to 13, wherein the number of carbon atoms in said counterpart is from 3 to 24.

15. The thickener according to any one of claims 1 to 14, wherein the counterpart is at least one selected from the group consisting of isostearic acid, lactic acid, lauric acid, myristic acid, palmitic acid, stearic acid, 12-hydroxystearic acid, behenic acid, and 3-(10-carboxydecyl)-1,1,1,3,5,5,5-heptamethyltrisiloxane.

16. The thickener according to any one of claims 1 to 4, wherein said water-soluble polymer includes an acidic functional group.

17. The thickener according to claim 16, wherein said water-soluble polymer is a copolymer of acrylic acid and an alkyl acrylate.

18. The thickener according to claim 16 or 17, wherein said water-soluble polymer is an acrylates/C10-30 alkyl acrylate crosspolymer.

19. The thickener according to any one of claims 16 to 18, wherein said counterpart is a cationic organic compound.

20. The thickener according to any one of claims 16 to 19, wherein said counterpart is distearyldimonium chloride.

21. The thickener according to any one of claims 1 to 20, wherein said water-soluble polymer and said counterpart form an ion pair.

22. The thickener according to any one of claims 1 to 21, wherein the content ratio of the counterpart is from 0.02 to 10 parts by mass with respect to 1 part by mass of said water-soluble polymer.

23. The thickener according to any one of claims 1 to 22, wherein the thickener is present in a polar solvent.

24. A thickened composition comprising:
the thickener for a non-aqueous system according to any one of claims 1 to 22; and
a polar solvent capable of dissolving said water-soluble polymer and said counterpart.

25. The composition according to claim 24, wherein the content by percentage of said thickener is from 0.1 to 15% by mass relative to the mass of the composition.

26. The composition according to claim 24 or 25, wherein said polar solvent is an organic solvent.

27. The composition according to any one of claims 24 to 26, wherein said polar solvent is at least one selected from the group consisting of ethanol, dipropylene glycol, butylene glycol, glycerin, polyethylene glycol, polypropylene glycol, octyl methoxycinnamate, octocrylene, homosalate, and octyl salicylate.

28. The composition according to any one of claims 24 to 27, wherein the content by percentage of said polar solvent is 20% by mass or greater relative to the mass of the composition.

29. The composition according to any one of claims 24 to 28, further comprising an oily component having mutual solubility to said polar solvent.

30. The composition according to claim 29, wherein said oily component is at least one selected from the group of consisting of hydrocarbon oils, ester oils, and silicone oils.

31. The composition according to claim 29 or 30, wherein the content by percentage of said oily component is 85% by mass or less relative to the mass of the composition.

32. The composition according to any one of claims 24 to 31, wherein, in a case where the composition includes water, the content by percentage of water is 10% by mass or less relative to the polar solvent.

33. The composition according to any one of claims 24 to 32, further comprising a UV absorber.

34. The composition according to claim 33, wherein said UV absorber is soluble in said oily component and/or said polar solvent.

35. The composition according to any one of claims 24 to 34, wherein the L* value in Lab color space is 90 or greater.

36. The composition according to any one of claims 24 to 35, wherein the viscosity is from 300 to 500,000 mPa·s.
